# EUROPEAN PATENT APPLICATION

(11) **EP 4 640 162 A1**
(43) Date of publication of application: **29.10.2025**
(21) Application number: 24172643.9
(22) Date of filing: 26.04.2024
(51) Int. Cl.: A61B 10/00, A61B 5/00, A61B 5/145, B01L 3/00

(54) **BIOLOGICAL FLUID COLLECTING DEVICE**

(71) Applicant: Université de Liège, 4000 Liège (BE)
(72) Inventor: CLOOTS, Rudi, 4000 Liège (BE); WISLET, Sabine, 4000 Liège (BE); TRAINA, Karl, 4000 Liège (BE)
(74) Representative: IP HILLS NV

(57) **Abstract**

Biological fluid collecting device comprising an upper surface and a lower surface, the lower surface being configured to face a user's skin during fluid collection, wherein the lower surface includes a fluid inlet, wherein the device further includes a fluid outlet, wherein the fluid inlet is fluidly connected to the fluid outlet via a duct, wherein a portion of the upper surface covering the duct is at least substantially translucent.

## Description

### Field of the Invention

The present invention generally relates to a biological fluid collecting device, in particular to a sweat collecting device for proteomic analysis.

### Background of the Invention

Biological fluid collecting devices are known, for example devices to collect bodily fluids, for example fluids produced by the human body, such as sweat, saliva or urine, or any other bodily fluids. The biological fluids are generally collected to analyse said fluids, for example to determine a volume produced over a predetermined period of time, to analyse a chemical composition of said fluids, and/or to control dehydration. It is further known that sweating can be a response to stress, physical exercise, depression or autonomous nervous system disorders. In particular in case of sweat collection, a perspiration rate can be determined. Abnormal perspiration rates can be associated with diseases such as hyperthyroidism, infections or neurological disorders. Sweat collecting devices are generally wearable or portable devices worn on the skin to allow continuous sweat collection and/or monitoring.

Biological fluid collecting devices are preferably configured to limit a risk of contamination of the collected sample, as well as to prevent sample evaporation. Thereto, the collecting devices need to be relatively simple to manipulate to prevent handling errors. At the same time, safety and comfort is important for the wearer of the biological fluid collecting device.

It has been found out that known biological fluid collecting devices, in particular wearable sweat collecting devices, suffer from a number of drawbacks, in particular when the collected sample is to be used for proteomic analyses. Sweat collecting devices generally make use of a dye, which may be included in a collection surface of the device, to visualize the collected sweat and assess a volume of said collected sweat. However, said dye may interfere with protein sweat content, thus making a reliable proteomic analysis very difficult or even impossible. A further drawback is that many sweat collecting devices are made of plastic material such as polystyrene, polyethylene or polypropylene, which are strongly hydrophobic and which may interact with hydrophobic proteins and peptides present in the sweat to be collected, resulting in a potential and unpredictable loss of peptides. As such, these materials are incompatible with collecting biological fluids for proteomic analyses including hydrophobic peptide analyses since it may lead to inaccurate measurements and/or potentially false conclusions. Additionally, biological fluid collecting devices are often single use devices, which makes them relatively expensive in substantially continuous monitoring. Moreover, said collecting devices are often relatively complex to assemble and/or to use, for example to retrieve the collected biological fluid.

### Summary of the Invention

It is therefore an aim of the present invention to solve or at least alleviate one or more of the above-mentioned problems. In particular, the invention aims at providing an improved biological fluid collecting device which is relatively easy to use and which is compatible with proteomic analyses of the collected biological fluid.

To this aim, according to a first aspect of the invention, there is provided a biological fluid collecting device having the features of claim 1. In particular, the biological fluid collecting device is configured to collect a sample of a biological fluid, such as for example sweat, saliva, urine or any other biological fluid. The biological fluid collecting device comprises an upper surface and a lower surface, the lower surface being configured to face a user's skin during fluid collection, while the upper surface opposite the lower surface is configured to face away from the skin during fluid collection. The lower surface includes a fluid inlet configured to allow a fluid to be collected to enter the fluid collecting device. The device further includes a fluid outlet. The fluid inlet is fluidly connected to the fluid outlet via a duct. A portion of the upper surface covering the duct is at least substantially translucent, translucent meaning that the upper surface lets light pass through, at least meaning that the upper surface may also be a substantially transparent upper surface through which an object is clearly and distinctively visible substantially without light scattering. Alternatively, an entire upper surface, i.e. not only the portion covering the duct, may be an at least substantially translucent upper surface. In an inventive way, the duct is delimited by substantially upstanding walls extending between the upper surface and the lower surface. As a result of the substantially upstanding walls, which are preferably substantially transverse to the upper surface and the lower surface, a cross-section of the duct may be substantially rectangular or at least substantially trapezoidal. Due to the different breaking angles of light through the substantially translucent upper surface, the duct including the substantially upstanding walls is visible when the duct is empty and is invisible when filled. As a result, there is no need to add a dye to make the filling of the duct visible. In this way, the collecting device becomes compatible with proteomic analyses. In other words, the collecting device is suitable for collecting a biological fluid on which proteomic analyses need to be performed.

The biological fluid collecting device may preferably be made of a single at least substantially translucent material. In analogy with the upper surface, at least substantially translucent means that the material is configured to let light pass through. The material may also, but need not, be a transparent material. Manufacturing the fluid collecting device of a single material can simplify the manufacturing process. The choice of an at least substantially translucent material is imposed by the need for an at least translucent upper surface allowing to see the filling of the duct.

The biological fluid collecting device may advantageously be made of a 3D printable resin composed of methacrylate-based monomers. The resin can preferably include 5 - 10 % of methacrylate monomers and more than 70% of dimethacrylates. In this way, the fluid collecting device can be manufactured by 3D printing, in particular by stereolithography 3D printing, which is a relatively efficient and cost-effective manufacturing process. A resin composed of methacrylate-based monomers is a photosensitive liquid resin hardening under ultraviolet light to form a hardened plastic. The collecting device may for example be made of a resin compatible with long-term skin contact and sterilization, such as for example Formlabs' BioMed Clear Resin. Further examples of biocompatible 3D printable resins may for example be found in "Biocompatible 3D printing resins for medical applications: A review of marketed intended use, biocompatibility certification, and post-processing guidance" in "Annals of 3D Printed Medicine, Volume 5, March 2022, 100044".

The upper surface of the biological fluid collecting device may preferably be substantially flat. A substantially flat surface can allow light to pass through said upper surface in a relatively uniform way over said upper surface, thus improving translucent properties of the surface. At the same time, a substantially flat surface is relatively easy to manufacture.

The substantially upstanding walls of the duct can advantageously be substantially monolithic with at least one of the upper surface and the lower surface, preferably with both the upper surface and the lower surface. More preferably, the entire biological fluid collecting device is a monolithic device. Monolithic parts, and in particular a one-piece device, can improve fluid tightness of the part or the device avoiding the need for assembly and/or fixation of different parts.

The substantially upstanding walls of the duct can preferably be substantially rigid. Substantially rigid upstanding walls can improve solidity of the device, which is more important when the device is reusable rather than when the device is configured to be single use only.

It is advantageous that the biological fluid collecting device is made of a hydrophilic material. A hydrophilic material can be defined as a material for which a contact angle of a droplet of liquid, for example water, on said material is inferior to 90° thus having a relatively high liquid - solid interaction. In this way, the device can enable the collection of biological fluids for proteomic analyses, for example metabolomic analyses, or ionic and hydrophobic peptide analyses or to perform mass spectroscopy and/or biomarkers identifications because protein desorption from the biological fluid collecting device is facilitated by the hydrophilic material. Hence, the number of proteins detected in a biological fluid collected by the present collecting device may be greater, such as 10%, preferably 20%, more preferably 30% greater than when collected by a prior art collecting device.

It may be preferred that the duct is a spiral duct. A spiral duct can have a relatively high length on a limited surface. Moreover, a spiral duct can allow a substantially central fluid inlet or outlet. A spiral duct has an additional advantage of being free of corners or other non-continuous portions which may complicate a fluid flow within the duct. Alternatively, other shapes for the duct may be used to an advantage.

The fluid outlet may preferably be formed by an opening in a lateral surface of the device. The lateral surface of the device may be substantially transverse to the upper surface and the lower surface of the device while connecting said upper surface and said lower surface. A lateral opening may improve comfort or convenience for the user wearing the device for biological fluid collection compared to a top or bottom opening. The lateral opening may at the same time facilitate fluid entrance and duct filling via the fluid inlet. The lateral opening may also be used for cleaning the device, in particular the duct, for potential re-use of the device. The fluid outlet may be sealable, for example by a cap, such as a silicone cap, or by any other sealing device, to allow conservation of the collected biological fluid within the duct before examination of the fluid.

More preferably, the fluid outlet may include a laterally protruding tube. Such a laterally protruding tube can facilitate connection to an extraction device configured to retrieve the collected fluid sample from the collecting device, for example by suction, to transfer the collected fluid into conventional fluid collection vials for further examination.

The fluid inlet may preferably be positioned substantially centrally on the lower surface. In this way, the biological fluid can be collected in a relatively evenly distributed way around the fluid inlet. In case there is a reason to assume that the biological fluid is mainly coming from a preferred direction, the fluid inlet may have a decentralized position on said lower surface.

The lower surface can advantageously be a substantially concave surface. The substantially concave shape of the lower surface may extend over substantially an entire lower surface of the device or over only a portion of the lower surface, for example over a substantially central portion of the lower surface, in particular over a portion of the lower surface directly under the duct. Thanks to this concave shape of the lower surface, a cavity is created between the skin of a user or wearer of the collecting device and the lower surface of the device when the device is worn on a user's skin. An outer edge of the lower surface, in particular an outer edge of the concave portion of the lower surface, can then be configured to engage the skin of the user delimiting said cavity. Said cavity can improve aspiration of a biological fluid towards the fluid inlet.

The lower surface can include a plurality of radial grooves extending from the fluid inlet. Said grooves can improve a flow or drain of the fluid to be collected towards the fluid inlet. Additionally, and/or alternatively, the lower surface may include a plurality of radial ribs extending from the fluid inlet. Said ribs can also improve a flow of the fluid towards the fluid inlet.

### Brief Description of the Drawings

Fig. 1 shows a perspective view of a preferred embodiment of a biological fluid collecting device according to the invention;
Fig. 2a, 2b and 2c show a transverse cross-sectional view and two top views respectively of the biological fluid collecting device of Figure 1;
Fig. 3a and 3b show a bottom view of the biological fluid collecting device of Figure 1.

### Detailed Description of Embodiment(s)

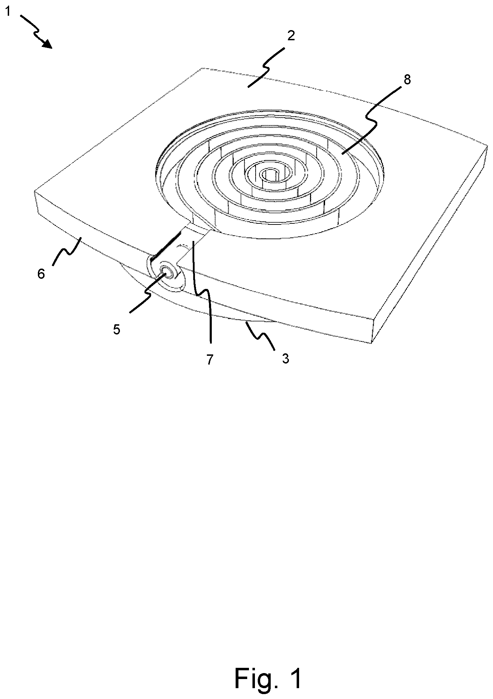

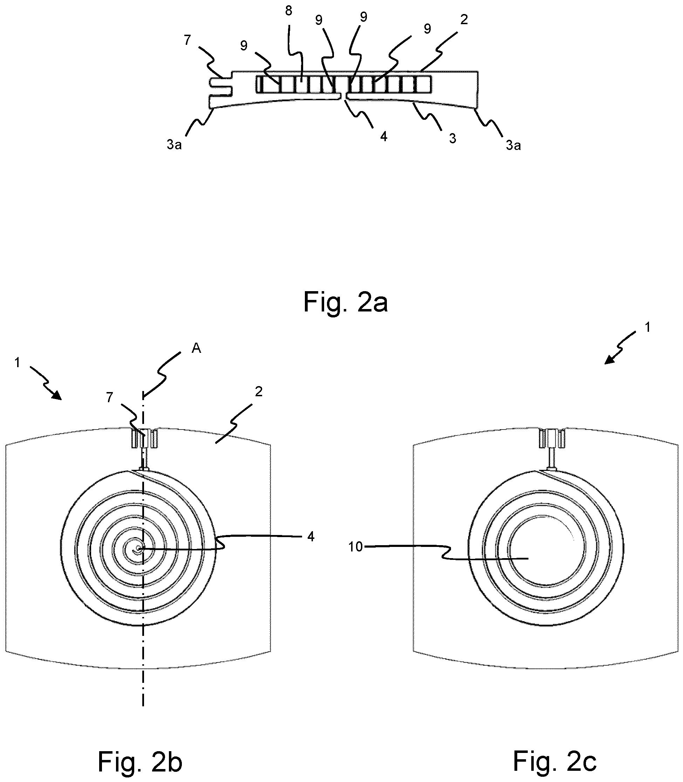

Figure 1 shows a perspective view of a preferred embodiment of a biological fluid collecting device 1 according to the invention. The biological fluid collecting device is configured to collect a biological fluid, such as for example sweat, saliva, urine or any other biological fluid. The collected fluid can then be transferred to a conventional vial for further analysis. Depending on which biological fluid is to be collected, the collecting device 1 can include attachment elements (not shown) allowing attachment of the device to a user, for example a band to wear the collecting device around an arm or a leg or any other body part when sweat collection is intended. When saliva is to be collected, the device can for example include a grip element allowing a user to hold the collecting device in one's mouth to suck on it like on a lollipop. The biological fluid collecting device 1 comprises an upper surface 2 and a lower surface 3. The lower surface 3 is configured to face a user's skin during fluid collection. The device 1 further includes a fluid inlet 4 (see Figure 2b) and a fluid outlet 5. The fluid outlet 5 is preferably formed by an opening in a lateral surface 6 of the device 1, the fluid outlet including for example a laterally protruding tube 7. The fluid inlet 4 is fluidly connected to the fluid outlet 5 via a duct 8, which can preferably have a spiral shape. A syringe or other extraction tool can for example be connected to the fluid outlet 5, in particular to the laterally protruding tube 7, which may be used to retrieve the collected fluid from the collecting device 1, either directly via said fluid outlet 5 or by pushing the fluid through the duct 8 and back out via the fluid inlet 4, in particular when only a limited quantity of a biological fluid has been collected. At the same time, the opening of the fluid outlet 5 can allow elimination of air from the duct 8 to allow the fluid to rise into the duct 8 by capillary action. Finally, the fluid outlet 5 may also be used for cleaning the collecting device 1 after the collected biological fluid has been retrieved. The upper surface 2 is an at least substantially translucent surface, in particular the portion covering the duct 8, and may also be a substantially transparent surface.

Figure 2a shows a transverse cross-sectional view according to axis A, and Figures 2b and 2c show two top views of the biological fluid collecting device of Figure 1, in which Figure 2b shows a substantially empty biological fluid collecting device and Figure 2c shows a biological fluid collecting device which is at least partially filled with a biological fluid. The upper surface 2 can preferably be a substantially flat surface, while the lower surface 3 may be a substantially concave surface, as can be seen in the cross-sectional view of Figure 2a. In an inventive way, the duct 8 is delimited by substantially upstanding walls 9 extending between the upper surface 2 and the lower surface 3. In case of a spiral duct, walls on either side of the duct laterally delimiting said duct may in fact be different portions of one and the same wall spiralling between the fluid inlet 4 and the fluid outlet 5. When these upstanding side walls 9 are mounted substantially transverse to the upper surface 2, a cross-section of the duct 8 has a substantially rectangular shape, which is different from prior art devices including tubes having substantially circular cross-sections. Thanks to the substantially translucent upper surface 2 in combination with the upstanding side walls extending between said upper surface 2 and said lower surface 3 resulting in said at least trapezoidal cross-sectional duct shape, the biological fluid collecting device 1 shows a particular and highly interesting feature which is illustrated in Figures 2b and 2c: when the duct is empty, i.e. when there is no biological fluid collected in the duct 8, then the duct 8 is visible through the upper surface 2, as shown in Figure 2b. However, when the duct 8 starts being filled with a collected biological fluid, the fluid collecting device 1 shows a kind of blind spot 10 which can expand from the fluid inlet 4 until where the duct is filled with said collected biological fluid. In fact, due to different breaking angles of light, the upstanding side walls 9 become undistinguishable from the collected biological fluid which is present in the duct 8. As a result, there is no need to add any colorant or dye to the biological fluid and/or to the duct, as done in prior art devices, to observe the filling of the duct 8 with the biological fluid being collected. This can allow proteomic analyses to be performed afterwards on the collected biological fluid without any bias caused by the addition of substances such as a dye.

For reasons of solidity of the device and/or ease of manufacturing, the substantially upstanding walls 9 of the duct can preferably be substantially rigid walls. More preferably, the substantially upstanding walls 9 of the duct 8 may be substantially monolithic with at least one of the upper surface 2 and the lower surface 3, or, most preferably, with both the upper surface 2 and the lower surface 3, thus providing a relatively solid device suitable for re-use after cleaning rather than a disposable device for single use only. The fluid collecting device can advantageously be 3D printed, preferably in a single substantially translucent material, such as for example in a 3D printable resin composed of methacrylate-based monomers. In an advantageous embodiment, the device is made of a hydrophilic material, which is for example composed of 5 - 10% of methacrylate monomers and of more than 70% of dimethacrylates, such as for example Formlabs' BioMed Clear Resin or any other suitable resin. BioMed Clear Resin is a hard, wear-resistant and transparent material for biocompatible applications requiring long-term contact with skin. This material is suitable for medical applications, as well as uses requiring low water absorption over time.

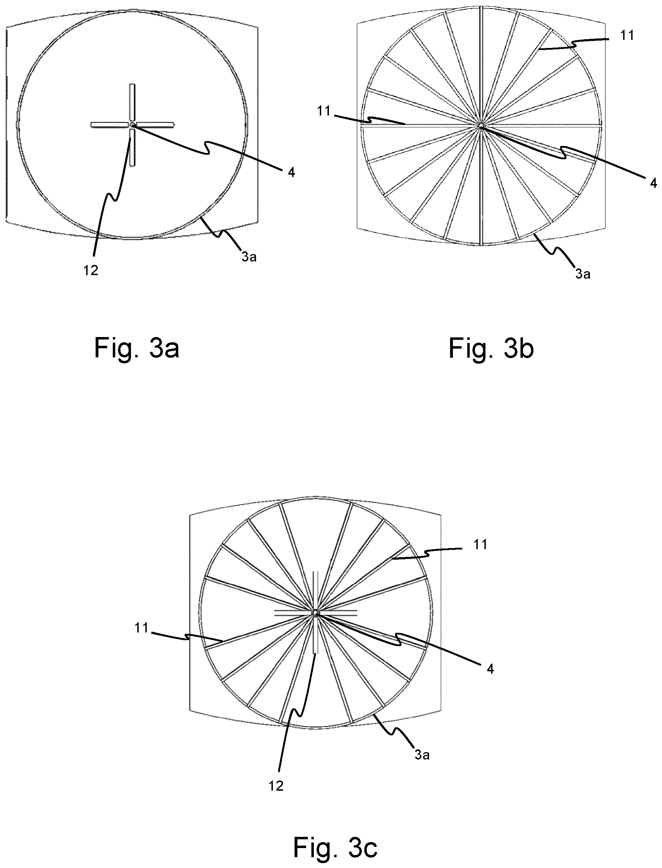

Figures 3a, 3b and 3c show a bottom view of different potential embodiments of the biological fluid collecting device 1 of Figure 1. Due to the substantially concave shape of the lower surface 4, as shown in Figure 2a, there may be an engagement, or even a kind of sealing, by an external edge 3a of the lower surface 3 on a user's skin when the device 1 is being worn while a cavity is created between the skin and the lower surface 3. The fluid inlet 4, formed by an opening in said lower surface 3, may be positioned substantially centrally on the lower surface 3. As a result, the fluid inlet 4 may then be then situated at a distance of the skin. To improve aspiration of a biological fluid towards the fluid inlet 4 of the collecting device 1, the lower surface may include a plurality of radial grooves 11 extending from the fluid inlet 4, as shown in Figure 3b and 3c. Said radial grooves may for example extend until an edge of the concave portion of the lower surface 3, which edge 3a may then be configured to be in engagement with a user's skin. Additionally, and/or alternatively, the lower surface 3 may include a plurality of radial ribs 12 extending from the fluid inlet 4, as shown in Figure 3a and 3c. Since the ribs protrude from the lower surface 4, they can, but need not, extend until said edge 3a configured to be in contact with a user's skin.

Although the present invention has been illustrated by reference to specific embodiments, it will be apparent to those skilled in the art that the invention is not limited to the details of the foregoing illustrative embodiments, and that the present invention may be embodied with various changes and modifications without departing from the scope thereof. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein. In other words, it is contemplated to cover any and all modifications, variations or equivalents that fall within the scope of the basic underlying principles and whose essential attributes are claimed in this patent application. It will furthermore be understood by the reader of this patent application that the words "comprising" or "comprise" do not exclude other elements or steps, that the words "a" or "an" do not exclude a plurality, and that a single element, such as a computer system, a processor, or another integrated unit may fulfil the functions of several means recited in the claims. Any reference signs in the claims shall not be construed as limiting the respective claims concerned. The terms "first", "second", third", "a", "b", "c", and the like, when used in the description or in the claims are introduced to distinguish between similar elements or steps and are not necessarily describing a sequential or chronological order. Similarly, the terms "top", "bottom", "over", "under", and the like are introduced for descriptive purposes and not necessarily to denote relative positions. It is to be understood that the terms so used are interchangeable under appropriate circumstances and embodiments of the invention are capable of operating according to the present invention in other sequences, or in orientations different from the one(s) described or illustrated above.

## Claims

1. Biological fluid collecting device comprising an upper surface and a lower surface, the lower surface being configured to face a user's skin during fluid collection, wherein the lower surface includes a fluid inlet, wherein the device further includes a fluid outlet, wherein the fluid inlet is fluidly connected to the fluid outlet via a duct, wherein a portion of the upper surface covering the duct is at least substantially translucent, and wherein the duct is delimited by substantially upstanding walls extending between the upper surface and the lower surface.

2. Biological fluid collecting device according to claim 1, wherein the device is made of a single at least substantially translucent material.

3. Biological fluid collecting device according to any of the preceding claims, wherein the device is made of a 3D printable resin composed of methacrylate-based monomers.

4. Biological fluid collecting device according to any of the preceding claims, wherein the upper surface is substantially flat.

5. Biological fluid collecting device according to any of the preceding claims, wherein the substantially upstanding walls of the duct are substantially monolithic with at least one of the upper surface and the lower surface.

6. Biological fluid collecting device according to any of the preceding claims, wherein the substantially upstanding walls of the duct are substantially rigid.

7. Biological fluid collecting device according to any of the preceding claims, wherein the device is made of a hydrophilic material.

8. Biological fluid collecting device according to any of the preceding claims, wherein the duct is a spiral duct.

9. Biological fluid collecting device according to any of the preceding claims, wherein the fluid outlet is formed by an opening in a lateral surface of the device.

10. Biological fluid collecting device according to any of the preceding claims, wherein the fluid outlet includes a laterally protruding tube.

11. Biological fluid collecting device according to any of the preceding claims, wherein the fluid inlet is positioned substantially centrally on the lower surface.

12. Biological fluid collecting device according to any of the preceding claims, wherein the lower surface is a substantially concave surface.

13. Biological fluid collecting device according to any of the preceding claims, wherein the lower surface includes a plurality of radial grooves extending from the fluid inlet.
